# EUROPÄISCHE PATENTANMELDUNG

(11) **EP 1 800 647 A1**
(43) Veröffentlichungstag der Anmeldung: **27.06.2007**
(21) Anmeldenummer: 06025535.3
(22) Anmeldetag: 11.12.2006
(51) Int. Cl.: A61K 8/06, A61K 8/92, A61Q 17/00

(54) **Emollient Mischung für kosmetische und pharmazeutische Formulierungen**

(30) Priorität: 20.12.2005 DE 102005061239
(71) Anmelder: Cognis IP Management GmbH, 40589 Düsseldorf (DE)
(72) Erfinder: Goget, Caroline, 75018 Paris (FR); Issberner, Ulrich, Ambler 19002 (US); Strauss, Gabriele, 40589 Düsseldorf (DE); Spörer, Roland, Klongtoey Bangkok 10110 (TH); Frisch, Albert, 67483 Edesheim (DE)

(57) **Zusammenfassung**

Gegenstand der Erfindung sind kosmetische oder pharmazeutische O/W-Emulsionen umfassend eine Ölphase und eine Wasserphase, wobei mindestens 40 Gew.-% der Ölphase eine Wachskomponente oder eine Gemisch aus Wachskomponenten ist sowie die Verwendung der Zusammensetzungen zur Hautpflege und zum Hautschutz von Personen, die Kontakt mit wässrigen Kühlschmierstoffen haben.

## Beschreibung

### Gebiet der Erfindung

Die Erfindung betrifft O/W-Emulsionen, deren Ölphase einen hohen Anteil an Wachskomponenten enthält sowie deren Verwendung zur Hautpflege und zum Hautschutz in speziellen Arbeitsbereichen.

### Stand der Technik

Moderne Produkte für den gewerblichen Hautschutz müssen auf den jeweiligen Arbeitsstoff abgestimmt sein. Im Falle von wassermischbaren Kühlschmierstoffen als Arbeitsstoff/Noxen wurden bisher Hautschutzprodukte auf Basis von Wasser-in-Öl **(W/O)** Emulsionen eingesetzt, die durch eine hohe Lipophilie einen natürlichen Schutz insbesondere gegen das Wasser darstellen. Jedoch stellt die Verwendung von Wasser-in-Öl-Emulsionen gerade für die in diesen Bereichen tätigen Beschäftigten (meist Männer) eine große "kulturelle" Hürde dar, da diese Art von Emulsionen nicht sofort einziehen und einen häufig fettigen und klebrigen Film hinterlassen.

Aufgabe der vorliegenden Erfindung war es, Formulierungen mit einem sehr guten und schnellen Einziehverhalten in die Haut bereitzustellen, die hohe Pflegeeigenschaften aufweisen und keinen klebrigen Film hinterlassen und sich als effektiver Schutz vor wässrigen Schadstoffen insbesondere Kühlschmierstoffen eignen.

### Beschreibung der Erfindung

Überraschenderweise wurde nun gefunden, dass diese Aufgabe durch O/W-Emulsionen mit einem relativ hohen Wachsanteil gelöst werden kann, deren äußere Phase Wasser ist. Die **O/W**-Emulsionen sind trotz des hohen Wachsgehaltes sehr stabil und ziehen sehr gut in die Haut ein.
Zudem konnte in verschiedenen Untersuchungen gezeigt werden, dass sie hervorragend gegen verschiedenste Kühlschmierstoffe, insbesondere wassermischbare Kühlschmierstoffe, schützen. Die Schutzwirkung ist dabei mindestens vergleichbar den herkömmlichen **W/O**-Emulsionen.

Gegenstand der Erfindung sind daher kosmetische oder pharmazeutische O/W-Emulsion umfassend eine Ölphase und eine Wasserphase, wobei mindestens 40 Gew.-% der Ölphase eine Wachskomponente oder eine Gemisch aus Wachskomponenten ist. In einer bevorzugten Ausführungsform der Erfindung liegt der Anteil der Wachskomponente oder eines Gemisches aus Wachskomponenten bei 50 - 70 Gew.-%, vorzugsweise 55 - 70 Gew.-%, bezogen auf die Ölphase. Vorzugsweise liegt der Schmelzpunkt wenigstens einer Wachskomponente bei wenigstens 30 °C, insbesondere 40 - 80°C und ganz besonders bevorzugt zwischen 40 - 70 °C.

Ein weiterer Gegenstand der Anmeldung ist die Verwendung der erfindunsgemäßen Zusammensetzungen zur Hautpflege und zum Hautschutz von Personen, die Kontakt mit Kühlschmierstoffen haben, insbesondere mit wassermischbaren Kühlschmierstoffen.

### Wachskomponenten

Unter dem Begriff Wachs werden üblicherweise alle natürlichen oder künstlich gewonnenen Stoffe und Stoffgemische mit folgenden Eigenschaften verstanden: sie sind von fester bis brüchig harter Konsistenz, grob bis feinkristallin, durchscheinend bis trüb und schmelzen oberhalb von 30 °C ohne Zersetzung. Sie sind schon wenig oberhalb des Schmelzpunktes niedrigviskos und nicht fadenziehend und zeigen eine stark temperaturabhängige Konsistenz und Löslichkeit. Erfindungsgemäß einsetzbar ist eine Wachskomponente oder ein Gemisch von Wachskomponenten, die bei 30 °C oder darüber schmelzen. Sie sind in den erfindungsgemäßen Zusammensetzungen in einer Gesamtmenge von wenigstens 40 Gew.-% bezogen auf die Ölphase enthalten. Besonders gut sind erfindungsgemäß Zusammensetzungen geeignet, deren Ölphase 50 - 70 Gew.-% Wachse enthält.

Als Wachse können erfindungsgemäß auch Fette und fettähnlichen Substanzen mit wachsartiger Konsistenz eingesetzt werden. Hierzu gehören u.a. Fette (Triglyceride), Mono- und Diglyceride, natürliche und synthetische Wachse, Fett- und Wachsalkohole, Fettsäuren, Ester von Fettalkoholen und Fettsäuren sowie Fettsäureamide, Paraffine oder Vaseline oder beliebige Gemische dieser Substanzen. Vorzugsweise wird ein Gemisch aus Wachskomponenten mit unterschiedlichen Schmelzpunkten eingesetzt, so dass sich auf der Haut eine "Schmelzkaskade" entwickelt. Dadurch ist ein nachhaltiger Pflege-Effekt gewährleistet und die Formulierung wird als besonders angenehm empfunden. Vorzugsweise liegt der Anteil der höher schmelzenden Wachse (40 - 80 °C) bei wenigstens 10 Gew.-%, insbesondere wenigstens 20 Gew.-% bezogen auf die Gesamtmenge der Wachskomponenten.

Unter **Fetten** versteht man Triacylglycerine, also die Dreifachester von Fettsäuren mit Glycerin. Bevorzugt enthalten sie gesättigte, unverzweigte und unsubstituierte Fettsäurereste. Hierbei kann es sich auch um Mischester, also um Dreifachester aus Glycerin mit verschiedenen Fettsäuren handeln. Erfindungsgemäß einsetzbar und als Konsistenzgeber besonders gut geeignet sind sogenannte gehärtete Fette und Öle, wie gehärtete Kern- und Fruchtöle, die durch Partialhydrierung gewonnen werden. Pflanzliche gehärtete Fette und Öle sind bevorzugt, z. B. gehärtetes Rizinusöl, Erdnußöl, Sojaöl, Rapsöl, Rübsamenöl, Baumwollsaatöl, Sojaöl, Sonnenblumenöl, Palmöl, Palmkernöl, Leinöl, Mandelöl, Maisöl, Olivenöl, Sesamöl, Kakaobutter und Kokosfett. Hierzu zählen beispielsweise die von der Cognis Deutschland GmbH & Co. KG vertriebenen Produkte Cegesoft^{®} SBE (Shea Butter) und Cegesoft® SH (Shorea Stenoptera).

Geeignet sind u.a. die Dreifachester von Glycerin mit C₁₂-C₆₀-Fettsäuren und insbesondere C₁₂-C₃₆-Fettsäuren. Hierzu zählt gehärtetes Rizinusöl, ein Dreifachester aus Glycerin und einer Hydroxystearinsäure, der beispielsweise unter der Bezeichnung Cutina^{®} HR im Handel ist. Ebenso geeignet sind Glycerintristearat, Glycerintribehenat (z. B. Syncrowax^{®} HRC), Glycerintripalmitat oder die unter der Bezeichnung Syncrowax^{®} HGLC bekannten Triglycerid-Gemische.

Als Wachskomponenten sind insbesondere Mono- und Diglyceride bzw. Mischungen dieser **Partialglyceride** einsetzbar. Zu den erfindungsgemäß einsetzbaren Glyceridgemischen zählen die von der Cognis Deutschland GmbH & Co. KG vermarkteten Produkte Novata^{®} AB und Novata^{®} B (Gemisch aus C₁₂-C₁₈-Mono-, Di- und Triglyceriden) sowie Cutina^{®} MD oder Cutina^{®} GMS (Glycerylstearat).

Mischester sowie Mischungen aus Mono-, Di- und Triglyceriden sind erfindungsgemäß bevorzugt geeignet, da sie eine geringere Neigung zur Kristallisation zeigen und somit die Performance der erfindungsgemäßen Zusammensetzung verbessern.

Zu den erfindungsgemäß bevorzugten Wachskomponenten zählen **Fettalkohole ,** z.B. die C₁₂-C₅₀-Fettalkohole, insbesondere die C₁₂-C₂₄-Fettalkohole und besonders bevorzugt C₁₆-C₂₄-Fettalkohole, die aus natürlichen Fetten, Ölen und Wachsen gewonnen werden, wie beispielsweise Myristylalkohol, 1-Pentadecanol, Cetylalkohol, 1-Heptadecanol, Stearylalkohol, 1-Nonadecanol, Arachidylalkohol, 1-Heneicosanol, Behenylalkohol, Brassidylalkohol, Lignocerylalkohol, Cerylalkohol oder Myricylalkohol. Erfindungsgemäß bevorzugt sind gesättigte unverzweigte Fettalkohole. Erfindungsgemäß einsetzbar sind auch Fettalkoholschnitte, wie sie bei der Reduktion natürlich vorkommender Fette und Öle wie z. B. Rindertalg, Erdnußöl, Rüböl, Baumwollsaatöl, Sojaöl, Sonnenblumenöl, Palmkernöl, Leinöl, Rizinusöl, Maisöl, Rapsöl, Sesamöl, Kakaobutter und Kokosfett anfallen. Es können aber auch synthetische Alkohole, z. B. die linearen, geradzahligen Fettalkohole der Ziegler-Synthese (Alfole^{®}) oder die teilweise verzweigten Alkohole aus der Oxosynthese (Dobanole^{®}) verwendet werden. Erfindungsgemäß bevorzugt geeignet sind C₁₄-C₁₈-Feftalkohole, die beispielsweise von der Cognis Deutschland GmbH unter der Bezeichnung Lanette^{®} 16 (C₁₆-Alkohol), Lanette^{®} 14 (C₁₄-Alkohol), Lanette^{®} O (C₁₆/C₁₈-Alkohol) und Lanette^{®} 22 (C₁₈/C₂₂-Alkohol) vermarktet werden. Fettalkohole verleihen den Zusammensetzungen ein trockeneres Hautgefühl und sind daher bevorzugt geeignet.

Als Wachskomponenten können auch C₁₄-C₄₀-**Fetsäuren** oder deren Gemische eingesetzt werden. Hierzu gehören beispielsweise Myristin-, Pentadecan-, Palmitin-, Margarin-, Stearin-, Nonadecan-, Arachin-, Behen-, Lignocerin-, Cerotin-, Melissin-, Eruca- und Elaeostearinsäure sowie substituierte Fettsäuren, wie z. B. 12-Hydroxystearinsäure, und die Amide oder Monoethanolamide der Fettsäuren, wobei diese Aufzählung beispielhaften und keinen beschränkenden Charakter hat.

Erfindungsgemäß verwendbar sind beispielsweise **natürliche pflanzliche Wachse**, wie Candelillawachs, Carnaubawachs, Japanwachs, Espartograswachs, Korkwachs, Guarumawachs, Reiskeimölwachs, Zuckerrohrwachs, Ouricurywachs, Montanwachs, Sonnenblumenwachs, Fruchtwachse wie Orangenwachse, Zitronenwachse, Grapefruitwachs, Lorbeerwachs (=Bayberrywax) und **tierische Wachse,** wie z. B. Bienenwachs, Schellackwachs, Walrat, Wollwachs und Bürzelfett. Im Sinne der Erfindung kann es vorteilhaft sein, hydrierte oder gehärtete Wachse einzusetzen. Zu den erfindungsgemäß verwendbaren natürlichen Wachsen zählen auch die **Mineralwachse**, wie z. B. Ceresin und Ozokerit oder die petrochemischen Wachse, wie z. B. Petrolatum, Paraffinwachse und Mikrowachse. Als Wachskomponente sind auch **chemisch modifizierte Wachse,** insbesondere die Hartwachse, wie z. B. Montanesterwachse, Sasolwachse und hydrierte Jojobawachse einsetzbar. Zu den **synthetischen Wachsen,** die erfindungsgemäß einsetzbar sind, zählen beispielsweise wachsartige Polyalkylenwachse und Polyethylenglycolwachse. Pflanzliche Wachse sind erfindungsgemäß bevorzugt. Auch Paraffine und Vaseline sind als Wachskomponenten einsetzbar.

Die Wachskomponente kann ebenso gewählt werden aus der Gruppe der **Wachsester** aus gesättigten und/oder ungesättigten, verzweigten und/oder unverzweigten Alkancarbonsäuren und gesättigten und/oder ungesättigten, verzweigten und/oder unverzweigten Alkoholen, aus der Gruppe der Ester aus aromatischen Carbonsäuren, Dicarbonsäuren, Tricarbonsäuren bzw. Hydroxycarbonsäuren (z. B. 12-Hydroxystearinsäure) und gesättigten und/oder ungesättigten, verzweigten und/oder unverzweigten Alkoholen, aus der Gruppe der mit Carbonsäuren veresterten Polyole, sowie ferner aus der Gruppe der Lactide langkettiger Hydroxycarbonsäuren. Beispiel solcher Ester sind die C₁₆-C₄₀-Alkylstearate, C₂₀-C₄₀-Alkylstearate (z. B. Kesterwachs^{®} K82H), C₂₀-C₄₀-Dialkylester von Dimersäuren, C₁₈-C₃₈-Alkylhydroxystearoylstearate oder C₂₀-C₄₀-Alkylerucate, Ester von Alkylcarbonsäuren mit Pentaerythrit, Dipentaerythrit, Tripentaerythrit und Tetrapenaterythrit. Ferner sind C₃₀-C₅₀-Alkylbienenwachs, Tristearylcitrat, Triisostearylcitrat, Stearylheptanoat, Stearyloctanoat, Trilaurylcitrat, Ethylenglycoldipalmitat, Ethylenglycoldistearat, Ethylenglykoldi(12-hydroxystearat), Stearylstearat, Palmitylstearat, Stearylbehenat, Cetylester, Cetearylbehenat und Behenylbehenat einsetzbar. Erfindungsgemäß bevorzugt ist der Einsatz von Wachsestern aus linearen, gesättigten C12-C24-Fettalkoholen und linearen, gesättigten C12-C24 Fettsäuren oder Hydroxyfettsäuren.

In einer bevorzugten Ausführungsform enthalten die erfindungsgemäßen Emulsionen eine Wachskomponente oder das Gemisch von Wachskomponenten ausgewählt aus der Gruppe der Fettalkohole, der gehärteten Mono-, Di- und Triglyceride oder einem Gemisch davon, der Fettsäuren, der natürliche pflanzlichen Wachse, der tierischen Wachse, der chemisch modifizierten Wachse, der synthetischen Wachse oder der Wachsester. In einer weiteren bevorzugten Ausführungsform wird als Wachskomponente(n) ein Wachsester in Kombination mit wenigstens einer weiteren Wachskomponente eingesetzt, vorzugsweise mit einem C12-C24-Fettalkohol. Ganz besonders bevorzugt ist wegen des positiven Schmelzkaskadeneffektes eine Mischung aus Wachsestern, Fettalkohol und Partialglyceriden, insbesondere C12-C24-Wachsestern, C12-C24-Fettalkoholen und C12-C24-Partialglyceriden, vorzugsweise in einer Menge von 50 - 100 Gew.-% bezogen auf Gesamtmenge der Wachskomponenten.

### Ölkörper

Die erfindungsgemäßen O/W-Emulsionen enthalten eine Ölphase, die wenigsten eine Ölkomponente, vorzugsweise jedoch ein Gemisch verschiedener Ölkomponenten enthält, die bei 20°C flüssig sind. Vorzugsweise beträgt der Anteil der Ölphase (inklusive Wachskomponenten, aber ohne oberflächenaktive Substanzen) bezogen auf die Gesamtzusammensetzung 15 - 35 Gew.-%, insbesondere 20 - 30 Gew.-% und besonders bevorzugt 25 - 30 Gew.-%. Als Ölkörper sind beispielhaft die nachstehend genannten Verbindungsklassen geeignet. Hierzu zählen u.a. Guerbetalkohole auf Basis von Fettalkoholen mit 6 bis 18, vorzugsweise 8 bis 10 Kohlenstoffatomen, flüssige Ester von linearen oder verzweigten, gesättigten oder ungesättigten C₆-C₂₂-Fettsäuren mit linearen oder verzweigten, gesättigten oder ungesättigten C₆-C₂₂-Fettalkoholen, insbesondere 2-Ethylhexanol. Beispielhaft seien genannt Hexyllaurat, Myristylisostearat, Myristyloleat, Cetylisostearat, Cetyloleat, Stearylisostearat, Stearyloleat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylisostearat, Behenyloleat, Erucylisostearat, Erucyloleat. Weitere geeignete Ester sind z.B. flüssige Ester von C₁₈-C₃₈-Alkylhydroxycarbonsäuren mit linearen oder verzweigten, gesättigten oder ungesättigten C₆-C₂₂-Fettalkoholen, flüssige Ester von linearen und/oder verzweigten, gesättigten oder ungesättigten Fettsäuren mit mehrwertigen Alkoholen (wie z. B. Propylenglycol, Dimerdiol oder Trimertriol) und/oder Guerbetalkoholen, Triglyceride oder Triglyceridmischungen, Mono-/Di-fTriglyceridmischungen, Ester von C₆-C₂₂-Feftalkoholen und/oder Guerbetalkoholen mit aromatischen Carbonsäuren, insbesondere Benzoesäure, Ester von C₂-C₁₂-Dicarbonsäuren mit linearen oder verzweigten, gesättigten oder ungesättigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare Dialkylcarbonate, Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z. B.
Finsolv® TN), lineare oder verzweigte, symmetrische oder unsymmetrische Dialkylether mit 6 bis 22 Kohlenstoffatomen pro Alkylgruppe, wie z. B. Di-n-octyl Ether (Cetiol® OE) oder Ringöffnungsprodukte von epoxidierten Fettsäureestern mit Polyolen, Kohlenwasserstoffe wie Paraffin- oder Mineralöle, Silikonöle und Oligo- oder Polyalphaolefine.

Emulsion die, dadurch gekennzeichnet sind, dass die Ölphase wenigstens eine Ölkomponente ausgewählt aus der Gruppe der Dialkylcarbonate, der Triglyceride, der Dialklyether, der C12-C40-Kohlenwasserstoffe oder einem Gemisch dieser Substanzen enthält, stellen eine bevorzugte Ausführungsform der Erfindung dar.

Die Dialkylcarbonate können symmetrisch oder unsymmetrisch, verzweigt oder unverzweigt, gesättigt oder ungesättigt sein und lassen sich nach Umesterungsreaktionen, die aus dem Stand der Technik hinlänglich bekannt sind, herstellen. Erfindungsgemäß besonders geeignet sind Dialkylcarbonate mit Alkylketten, die 6 bis 24 Kohlenstoffatome aufweisen, insbeondere Di-n-octylcarbonat oder Di-(2-ethylhexyl)carbonat oder ein Gemisch dieser Substanzen. Unter diesen ist das Di-n-Octylcarbonat bevorzugt.

Die erfindungsgemäß einsetzbaren Kohlenwasserstoffe weisen eine Kettenlänge von 8 bis 40 C-Atomen aus. Sie können verzweigt oder unverzweigt sein, gesättigt oder ungesättigt. Unter diesen sind verzweigte, gesättigte C8-C40-Alkane bevorzugt. Es können sowohl Reinsubstanzen eingesetzt werden als auch Substanzgemische. Üblicherweise handelt es sich um Substanzgemische verschiedener isomerer Verbindungen. Zusammensetzungen, die Alkane mit 10 bis 30, vorzugsweise 12 bis 20, und besonders bevorzugt 16 bis 20 Kohlenstoffatome aufweisen, sind besonders geeignet, und unter diesen ein Gemisch aus Alkanen, welches wenigstens 10 Gew.-% verzweigte Alkane bezogen auf die Gesamtmenge der Alkane enthält. Vorzugsweise handelt es sich um verzweigte, gesättigte Alkane. Besonders gut geeignet sind Gemische aus Alkanen ist, welche mehr als 1 Gew.-% 5,8-Diethyldodecan und/oder mehr als 1 Gew.-% Didecen enthalten.

### Oberflächenaktive Substanzen

Eine weitere bevorzugte Ausführungsform der erfindungsgemäßen Zusammensetzung enthält zusätzlich wenigstens einen grenzflächenaktiven Stoff aus der Gruppe der nichtionischen und/oder anionischen Tenside. Partialglyceride, Glyceride und Fettalkohole werden erfindungsgemäß nicht zu den oberflächenaktiven Stoffen gerechnet; sie gehören erfindungsgemäß zur Gruppe der Wachse. Der Gehalt des oberflächenaktiven Stoffs hängt von der Art der Formulierung ab, übersteigt aber üblicherweise 10 Gew.-% nicht. Der bevorzugte Gehalt liegt zwischen 0,5 -10 Gew.-%, vorzugsweise 0,5 - 5 Gew.-% und insbesondere 1- 3 Gew.-% bezogen auf die Gesamtzusammensetzung.

Typische Beispiele für **nichtionische grenzflächenaktive Stoffe** sind Fettalkoholpolyglycolether, Polyglycerinester, Alkylphenolpolyglycolether, Fettsäurepolyglycolester, Fettsäureamid-polyglycolether, Fettaminpolyglycolether, alkoxylierte Mono-/Di-/Triglyceride, Mischether bzw. Mischformale, Alk(en)yloligoglykoside bzw. Glucoronsäurederivate - gegebenenfalls partiell oxidiert, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. In einer besonders bevorzugten Ausführungsform ist der nichtionische grenzflächenaktive Stoff ausgewählt aus der Gruppe der Alk(en)yloligoglycoside.

Alk(en)yloligoglykoside stellen bekannte nichtionische Tenside dar, die der Formel (I) folgen,

**R¹O-[G]ₚ** (I)

in der R¹ für einen Alk(en)ylrest, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Stellvertretend für das umfangreiche Schrifttum sei hier auf die EP 0301298 A1 und WO 90103977 A verwiesen.

Die Alk(en)yloligoglykoside können sich von Aldosen bzw. Ketosen mit 5 oder 6 Kohlenstoffatomen, vorzugsweise der Glucose, ableiten. Die bevorzugten Alkyloligoglykoside sind somit Alkyloligoglucoside. Die Indexzahl p in der allgemeinen Formel (I) gibt den Oligomerisierungsgrad (DP-Grad) an, d. h. die Verteilung von Mono- und Oligoglykosiden, und steht für eine Zahl zwischen 1 und 10. Während p in einer gegebenen Verbindung stets ganzzahlig sein muss und hier vor allem die Werte p = 1 bis 6 annehmen kann, ist der Wert p für ein bestimmtes Alk(en)yloligoglykosid eine analytisch ermittelte rechnerische Größe, die meistens eine gebrochene Zahl darstellt. Vorzugsweise werden Alkyloligoglykoside mit einem mittleren Oligomerisierungsgrad p von 1,1 bis 3,0 eingesetzt. Aus anwendungstechnischer Sicht sind solche Alk(en)yloligoglykoside bevorzugt, deren Oligomerisierungsgrad kleiner als 1,7 ist und insbesondere zwischen 1,2 und 1,4 liegt. Erfindungsgemäß bevorzugt werden Alkyloligoglykoside verwendet, in denen sich der Rest R¹ von primären Alkoholen mit 12 bis 24 Kohlenstoffatomen, insbesondere 16 bis 18 Kohlenstoffatomen ableitet. Es können auch technische Gemische der Alkohole eingesetzt werden. Erfindungsgemäß besonders bevorzugt ist der Einsatz von Emulgade® PL 68/50 (Cognis Deutschland GmbH & Co. KG), ein Gemisch aus Ceteaylalkohol und Cetearylglcosid mit ca. 50 Gew.-% Ceteraylglucosid. Das Alk(en)yloligoglycosid oder Gemisch aus Alk(en)yloligoglycosiden wird vorzugsweise in Mengen von 0,1 - 5,0 Gew.-% bezogen auf die Gesamtzusammensetzung eingesetzt.

Als oberflächenaktive Stoffe können auch anionische, kationische und/oder amphotere bzw. zwitterionische Tenside/Emulgatoren oder ein Gemisch dieser Tenside/Emulgatoren in den erfindungsgemäßen Zusammensetzungen enthalten sein.

Typische Beispiele für **anionische Tenside** sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Unter den anionischen Tensiden sind diejenigen bevorzugt, die besonders mild und hautfreundlich sind. Hierzu gehören insbesondere Sulfosuccinate, Acyllactylate, Acyltartrate, Acylglutamate und Acylaspartate, Proteinfettsäurekondensate (insbesondere pflanzliche Produkte auf Weizenbasis) und Alkyl(ether)phosphate.
Erfindungsgemäß besonders bevorzugt ist der Einsatz von Amphisol® K (INCI: Natrium Cetyl Phosphat), vorzugsweise in Mengen von 0,1 - 2,0 Gew.-%, insbesondere 0,1 -1,0 Gew.-% bezogen auf die Gesamtzusammensetzung.

Typische Beispiele für **kationische Tenside** sind quartäre Ammoniumverbindungen, wie beispielsweise das Dimethyldistearylammoniumchlorid, und Esterquats, insbesondere quatemierte Fettsäuretrialkanolaminestersalze. Typische Beispiele für **amphotere bzw. zwitterionische Tenside** sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten auf diesem Gebiet verwiesen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

Eine weitere bevorzugte Zusammensetzung ist dadurch gekennzeichnet, dass sie einen Gehalt an folgenden Komponenten aufweist:
(a) 15 - 35 Gew.-% einer Ölphase, die 40 - 70 Gew.-% eines Gemisches and Wachskomponenten enthält, umfassend wenigstens C16-C24-Fettalkohole und der Wachsester aus C12-C24-Fettalkoholen und C12-C24-Fettsäuren
(b) 0,5 - 5 Gew.-% eines nichtionischen Tensids ausgewählt aus der Gruppe der Alk(en)yloligoglycoside
(c) 50 - 80 Gew.-% Wasser
(d) und optional weitere Hilfs- und Zusatzstoffe.

### Weitere fakultative Hilfs- und Zusatzstoffe

Je nach Applikationszweck enthalten die kosmetischen Formulierungen eine Reihe weiterer Hilfs- und Zusatzstoffe wie beispielsweise Verdickungsmittel, Überfettungsmittel, Stabilisatoren, Polymere, Lecithine, Phospholipide, biogene Wirkstoffe, UV-Lichtschutzfaktoren, Antioxidantien, Filmbildner, Quellmittel, Insektenrepellentien, Hydrotrope, Solubilisatoren, Konservierungsmittel, Parfümöle, Farbstoffe etc., die nachstehend exemplarisch aufgelistet sind. Die Mengen der jeweiligen Zusätze richten sich nach der beabsichtigten Verwendung.

Als **Verdickungsmittel** eignen sich beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, Polyvinylalkohol, Polyvinylpyrrolidon und Bentonite wie z. B. Bentone^{®} Gel VS-5PC (Rheox). Elektrolyte wie Kochsalz und Ammoniumchlorid sind im Allgemeinen mit erfindungsgemäßen Zusammensetzungen in höheren Konzentrationen nicht kompatibel.

Unter **UV-Lichtschutzfaktoren** sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z. B. Wärme wieder abzugeben. UV-B-Filter können öllöslich oder wasserlöslich sein. Als typische UV-A-Filter kommen insbesondere Derivate des Benzoylmethans in Frage. Die UV-A und UV-B-Filter können selbstverständlich auch in Mischungen eingesetzt werden, z.B. Kombinationen aus den Derivaten des Benzoylmethans, z. B. 4-tert.-Butyl-4'-methoxydibenzoylmethan (Parsol^{®} 1789) und 2-Cyano-3,3-phenylzimtsäure-2-ethyl-hexylester (Octocrylene) sowie Estern der Zimtsäure, vorzugsweise 4-Methoxyzimtsäure-2-ethylhexylester und/oder 4-Methoxyzimtsäurepropylester und/oder 4-Methoxyzimtsäureisoamylester. Häufig werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert. Neben den genannten löslichen Stoffen kommen auch unlösliche Lichtschutzpigmente, nämlich feindisperse Metalloxide in Frage. Beispiele für geeignete Metalloxide sind insbesondere Zinkoxid und Titandioxid. Neben den beiden vorgenannten Gruppen primärer Lichtschutzstoffe können auch sekundäre Lichtschutzmittel vom Typ der **Antioxidantien** eingesetzt werden, die die photochemische Reaktionskette unterbrechen, welche ausgelöst wird, wenn UV-Strahlung in die Haut eindringt.

Unter **biogenen Wirkstoffen** sind beispielsweise Tocopherol, Tocopherolacetat, Tocopherolpalmitat, Ascorbylpalmitat, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z. B. Prunusextrakt, Bambaranussextrakt und Vitaminkomplexe zu verstehen.

Als **Insekten-Repellentien** kommen beispielsweise N,N-Diethyl-m-toluamid, 1,2-Pentandiol oder 3-(N-n-Butyl-N-acetyl-amino)-propionsäureethylester), welches unter der Bezeichnung Insect Repellent^{®} 3535 von der Merck KGaA vertrieben wird, sowie Butylacetylaminopropionate in Frage.

Zur Verbesserung des Fließverhaltens können ferner **Hydrotrope,** wie beispielsweise Ethanol, Iso-propylalkohol, oder Polyole eingesetzt werden. Polyole, die hier in Betracht kommen, besitzen vorzugsweise 2 bis 15 Kohlenstoffatome und mindestens zwei Hydroxylgruppen.

Als **Konservierungsmittel** eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

Als **Parfümöle** seien genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen, Harzen und Balsamen. Weiterhin kommen tierische Rohstoffe, wie beispielsweise Zibet und Castoreum sowie synthetische Riechstoffverbindungen vom Typ der Ester, Ether, Aldehyde, Ketone, Alkohole und Kohlenwasserstoffe in Frage.

### Beispiele:

**Tabelle 1**

| **Hautschutz - Rezepturen** | **1** | **2** | **3** | **4** | **5** |
|---|---|---|---|---|---|
| **Handelsname (INCI)** | | | | | |
| Emulgade® PL 68/50 (Cetearyl Glucoside und Cetearyl Alkohol) | 5 | 5 | 5 | 5 | 5 |
| Amphisol® K (Kalium Cetyl Phosphate) | | | | 0,3 | 0,3 |
| Cutina® MD (Glyceryl Stearate) | | | | 2 | 2 |
| Cetiol® CC (Dicaprylyl Carbonate) | 5 | 5 | 5 | 5 | 5 |
| Myritol® 331 (Cocoglyceride) | 2 | 2 | 2 | 2 | 2 |
| Cetiol® S (Diethylhexylcyclohexan) | | | | | |
| Lunacera® M (Ozokerite) | | | | | |
| Vaseline, Petrolatum | | | | | |
| Cegesoft® PS6 (Olus) | 3 | 3 | 3 | 2 | 2 |
| Cegesoft® SBE (Butyrospermum Parkii) | 1 | 1 | | | |
| Cegesoft® SH (Shorea Stenoptera) | 2 | 2 | 1 | 1 | 1 |
| Cetiol® MM (Myristyl Myristat) | 5 | 5 | 5 | 5 | 5 |
| Novata® B (Mono-, Di-, Triglyceride, C12 - C18) | 5 | 5 | 2,5 | 2,5 | 2,5 |
| Bienenwachs 8100, Cera alba | 2 | 2 | 1 | 6 | 6 |
| Wasser, VE | 69,13 | 64,5 | 70 | 53,7 | 53,7 |
| Zincum® N 29 (Zink Stearat) | | | | | |
| Dry Flo Plus® (Aluminium Stärke Octenyl Succinat) | | | | | |
| Microwax, Ozokerite | | | | | |
| Copherol® F 1300 (Tocopherol) | 0,2 | | | | |
| Solbrol® ICG (Jodopropynyl butylcarbamat) | 0,05 | | | | |
| Neo Dragocid® Liquid (Triethylen Glycol, Imidazolidinyl Urea, Methylparaben, Propylparaben, Dehydracetsäure) | 0,6 | | | | |
| Ascorbylpalmitat | 0,02 | | | | |
| Hydagen® CMF (10%); (Chitosan Glycolat) | | | | 10 | |
| Hydagen® HCMS-LA (Chitosan Lactat) | | | | | 10 |
| Glycerin | | 5 | 5 | 5 | 5 |
| Uniphen® P 23 (Phenoxyethanol, Methyl-, Ethyl-, Propyl-, Butylparabene) | | 0,5 | 0,5 | 0,5 | 0,5 |

**Tabelle 2:**

| **Hautschutz - Rezepturen** | **6** | **7** | **8** | **9** | **10** | **11** |
|---|---|---|---|---|---|---|
| Handelsname (INCI) | | | | | | |
| Emulgade® PL 68/50 (Cetearyl Glucoside und Cetearyl Alcohol) | 5 | 5 | 5 | 5 | 5 | 5 |
| Amphisol® K (Kalium Cetyl Phosphate) | 0,3 | 0,3 | 0,3 | 0,3 | 0,3 | 0,3 |
| Cutina® MD (Glyceryl Stearat) | 2 | 2 | 2 | 2 | 2 | 2 |
| Cetiol® CC (Dicaprylyl Carbonat) | 5 | 5 | 5 | 5 | 5 | 5 |
| Myritol® 331 (Cocoglyceride) | | | | | | 2 |
| Cetiol® S (Diethylhexylcyclohexan) | 3 | 3 | 3 | 3 | 3 | |
| Lunacera® M (Ozokerite) | | | | | | |
| Vaseline, Petrolatum | 6 | 6 | 6 | 6 | 6 | |
| Cegesoft® PS6 (Olus) | 2 | 2 | 2 | 2 | 2 | 2 |
| Cegesoft® SBE (Butyrospermum Parkii) | | | | | | |
| Cegesoft® SH (Shorea Stenoptera) | | | | | | 1 |
| Cetiol® MM (Myristyl Myristat) | 5 | 5 | 5 | 5 | 5 | 5 |
| Novata® B (Mono-, Di-, Triglyceride, C12 - C18) | | | | | | 2,5 |
| Bienenwachs 8100, Cera alba | | | | 2 | | 6 |
| Wasser, VE | 71,7 | 69,7 | 69,7 | 69,7 | 69,7 | 64,2 |
| Zincum® N 29 (Zink Stearat) | | 2 | | | | |
| Dry Flo Plus® (Aluminium Stärke Octenyl Succinat) | | | 2 | | | |
| Microwax, Ozokerite | | | | | 2 | |
| Copherol® F 1300 (Tocopherol) | | | | | | |
| Solbrol® ICG (Jodopropynyl butylcarbamate) | | | | | | |
| Neo Dragocid® Liquid (Triethylene Glycol, Imidazolidinyl Urea, Methylparaben, Propylparaben, Dehydracetsäure) | | | | | | |
| Ascorbylpalmitat | | | | | | |
| Hydagen® CMF (10%); (Chitosan Glycolat) | | | | | | |
| Hydagen® HCMS-LA (Chitosan Lactat) | | | | | | |
| Glycerin | | | | | | 5 |
| Uniphen® P 23 (Phenoxyethanol, Methyl-, Ethyl-, Propyl-, Butylparabene) | | | | | | |

### Prüfung der erfindunsgemäßen Rezepturen im Vergleich zu handelsüblichen Produkten

Die erfindungsgemäßen Formulierungen wurden bezüglich Pflege- und Schutzwirkung gegen wasser- bzw. fettlösliche Noxen im standardisierten BUS-Hautschutzmodell (Bovine Udder Test) auf follikelhaltiger und nicht-follikelhaltiger Haut untersucht: Förster Th., Pittermann W., Schmitt M., Kietzmann M. (1999), "Skin penetration properties of cosmetic formulations using a perfused bovine udder model"; J. Cosmet. Sci. 50, 147-157**;** Jackwerth B., Pittermann W., Kietzmann M. (1996): "Bovine udder skin model (BUS): Innovatives in-vitro-Modell zur Penetration, Resorption und Imitationswirkung kosmetischer Stoffe und Formulierungen"; Parfümerie und Kosmetik 77, 37-40**;** Kietzmann M.; Löscher W., Arens D., Maaß P., Lubach D. (1993): "Perfused bovine udder as an in-vitro model of percutaneous drug absorption. Skin viability and percutaneous absorption of dexamethasone, benzoyl proxide and etofenamate"; J. Pharm. Toxicol. Meth. 30, 75-84**;** Pittermann W., Hörner V., Förster Th., Kietzmann M. (1997): "Use of natural and artificial skin models in cosmetic research"; SÖFW-Journal 123, 666-670**;** Pittermann W., Jackwerth B., Schmitt M. (1997): "The isolated perfused bovine udder skin model: A new in-vitro model for the assessment of skin penetration and irritation"; Invitro Toxicology 10,17-21.

Als Noxen wurden hierfür SDS (Natriumdodecylsulfat), Toluol und wassermischbare Kühlschmierstoffe eingesetzt. Die erfindungsgemäßen Rezepturen wurden bezüglich ihrer Wirksamkeit im Vergleich zu zwei handelsüblichen Produkten geprüft: Saniwip®, eine handelsübliche Stearatcreme, z.B.
Stokolan®, ein O/W-Hautpflegemittel. Als Standard mit 100%-iger Schutzwirkung (=Positivkontrollen) dienten Vaseline (DAB) oder Wollfettwachs (DAB).

Das Prinzip ist a) die Prüfung der Hautverträglichkeit der Produkte, b) das Irritationspotential der Noxen und c) die Noxeneffekte nach Vorbehandlung der Haut durch die Prüfsubstanzen (Produkte) in Abhängigkeit zur Expositionszeit (15, 60 und 300 Minuten nach Noxenapplikation). Verglichen wird das Schutzpotential, das sich nach einer Einwirkzeit von 15 Minuten einstellt, und je nach Qualität bis zu 5 Stunden andauert. Je früher der Schutz effektiv wird, umso weniger kann die Noxe die Haut penetrieren und zelluläre Reaktionen induzieren.

Das Prüfdesign erlaubt den vergleichenden Einsatz von 2 Prüfsubstanzen und einer Noxe unter gleichen experimentellen Bedingungen. Auf diese Weise kann der Einfluss der qualitativ unterschiedlichen Vorbehandlung auf Schädigung durch die Noxe gemessen werden. Als Bewertungsmaßstab wird die prozentuale Reduktion der Noxenschädigung verwendet.

Insgesamt wurden zwei Formulierungen mit zwei Marktprodukten für Hautpflege bzw. Hautschutz in ihrer Wirksamkeit gegen SDS und Toluol (wasser- bzw. fettlösliche Noxe) geprüft. Toluol und SDS verursachen biochemisch nachweisbare Hautschäden nach einer Expositionszeit von 60 und 300 Minuten. Nicht immer ist nach 15 Minuten ist eine ausreichende Schädigung erreicht und daher auch kein Schutzpotential durch die Formulierungen bzw. Produkte nachzuweisen. Beide Noxen induzierten im Wesentlichen zytotoxische Schäden. Generell ist das Hautschutzpotential gegen fettlösliche Noxen, wie Toluol, vergleichsweise weniger gut ausgebildet als gegen SDS.

### Hautschutzapplikation

**Follikuläre/nicht-follikuläre Haut:** Die Expositionszeit des einzelnen Produkts (erfindungsgemäße Rezepturen sowie Saniwip® und Stokolan® als Vergleich) betrug 0,25h (Beginn der Noxenapplikation) (Applikationsmenge 2g/100cm²), anschließend wurde die produktbehandelte Stelle mit der Noxe (z.B. *Toluol*-getränktes Textil) für 0,25h bedeckt, die Noxe nach 0,25h mit dem Textil entfernt und die erste Hautbiopsie (0,25h) gestanzt. 1,0h bzw. 5,0h nach Beginn der Noxenapplikation wurde die 2. bzw. 3. Biopsie gewonnen. Anzahl der Untersuchungen / Prüfsubstanz: Follikuläre Haut (n=4 Euter, 72 Ganzhautbiopsien); Nicht-follikuläre Haut (n=2 Euter; 24 Ganzhautbiopsien).

Die Ergebnisse sind in nachfolgenden Tabellen zusammengefasst. Der erste Wert in den Tabellen bezieht sich auf follikuläre Haut, der Wert in Klammern auf nicht-follikuläre Haut.

### 1. Erfindungsgemäße Rezeptur im Vergleich zu Saniwip® (Tabelle 3-6); Noxe = Toluol oder SDS

**Tabelle 3:**

| Schutzwirkung gegen Schädigung durch Toluol: Follikuläre Haut (nicht-follikuläre Haut) | | | |
|---|---|---|---|
| Prüfsubstanz | **Exposition: 0,25h** | Exposition: 1,0h | Exposition:5,0h |
| Beispiel 11 | Nicht bestimmt | 59 % (70 %) | 42 % (9 %) |
| ***Saniwip®*** | Nicht bestimmt | 52 % (44 %) | 33 % (13 %) |

| | | | |
|---|---|---|---|
| Verglichen mit den beiden Positivkontrollen (Vaseline und Wollfettwachs = 100%) liegt die Schutzwirkung Beispiel 11 und *Saniwip®* bei ca. 59 % bzw. 42 %. | | | |

**Tabelle 4**

| Schutzwirkung gegen Schädigung durch *Toluol:* Follikuläre Haut (nicht-follikuläre Haut) | | | |
|---|---|---|---|
| Prüfsubstanz | **Exposition: 0,25h** | **Exposition: 1,0h** | **Exposition: 5,0h** |
| Beispiel 4 | Nicht bestimmt | 42 % (75 %) | 46 % (58 %) |
| ***Saniwip®*** | Nicht bestimmt | 9 % (30 %) | 43 % (29 %) |

| | | | |
|---|---|---|---|
| Verglichen mit den beiden Positivkontrollen (Vaseline und Wollfettwachs = 100%) liegt die Schutzwirkung von Beispiel 4 und *Saniwip®* bei ca. 99 % bzw. 44 %. | | | |

**Tabelle 5**

| Schutzwirkung gegen Schädigung durch *SDS* (10 Gew.-%) Follikuläre Haut (nicht-follikuläre Haut) | | | |
|---|---|---|---|
| Prüfsubstanz | **Exposition: 0,25h** | **Exposition: 1,0h** | **Exposition: 5,0h** |
| Beispiel 11 | Nicht bestimmt | 70 % (80 %) | 100 % (57 %) |
| ***Saniwip®*** | Nicht bestimmt | 65 % (69 %) | 54 % (66 %) |

| | | | |
|---|---|---|---|
| Verglichen mit einer Positivkontrolle (Vaseline: 100%) liegt die Schutzwirkung von Beispiel 11 und *Saniwip®* bei ca. 103 % bzw. 100 %. | | | |

**Tabelle 6**

| Schutzwirkung gegen Schädigung (=100%) durch *SDS* (10 Gew.-%) Follikuläre Haut (nicht-follikuläre Haut) | | | |
|---|---|---|---|
| Prüfsubstanz | **Exposition: 0,25h** | **Exposition: 1,0h** | **Exposition: 5,0h** |
| Beispiel 4 | 100 % (nicht bestimmt) | 86 % (100 %) | 85 % (81 %) |
| ***Saniwip®*** | 43 % (nicht bestimmt) | 46 % (23 %) | 40 % (36 %) |

| | | | |
|---|---|---|---|
| Verglichen mit einer Positivkontrolle (Vaseline = 100%) liegt die Schutzwirkung von Beispiel 4 und *Saniwip®* bei ca. 136 % bzw. 62 %. | | | |

### 2. Erfindungsgemäße Rezeptur im Vergleich zu Stokolan® (Tabelle 7-8); Noxe = Toluol oder SDS

**Tabelle 7**

| Schutzwirkung gegen Schädigung (=100%) durch *SDS* (10 Gew.-%) Follikuläre Haut (nicht-follikuläre Haut) | | | |
|---|---|---|---|
| Prüfsubstanz | **Exposition:0,25h** | **Exposition: 1,0h** | **Exposition:5,0h** |
| Beispiel 5 | Nicht bestimmt (70 %) | 54 % (80 %) | 53 % (66 %) |
| ***Stokolan®*** | Nicht bestimmt (0 %) | 37 % (23 %) | 41 % (33 %) |

| | | | |
|---|---|---|---|
| Verglichen mit einer Positivkontrolle (Vaseline: 100%) liegt die Schutzwirkung von Beispiel 5 und *Stokolan®* bei ca. 100 % bzw. 44%. | | | |

**Tabelle 8**

| Schutzwirkung gegen Schädigung (=100%) durch Toluol Follikuläre Haut (nicht-follikuläre Haut) | | | |
|---|---|---|---|
| **Prüfsubstanz** | Exposition:0,25h | Exposition: 1,0h | Exposition:5,0h |
| Beispiel 5 | 53 % (nicht bestimmt) | 50 % (45 %) | 55 % (48 %) |
| **Stokolan®** | 0 % (nicht bestimmt) | 24 % (0 %) | 31 % (23 %) |

| | | | |
|---|---|---|---|
| Verglichen mit den beiden Positivkontrollen (Vaseline, Wollwachssalbe = 100%) liegt die Schutzwirkung von Beispiel 5 und *Stokolan®* bei ca. 70 % bzw. 18 %. | | | |

### 3. Ergebnisse und Zusammenfassung der aus den Tabellen 3 - 8

### Tabelle 9: Schutzwirkung gegen Schädigung durch Toluol oder SDS

Reduktion der Noxenschädigung <40%: nicht ausreichend; = Pflegeeffekt, aber kein Schutzeffekt

Reduktion der Noxenschädigung <50%: befriedigend; = Pflegeeffekt; evtl. Schutzeffekt

Reduktion der Noxenschädigung >50%: gut

Reduktion der Noxenschädigung >60%: sehr gut

Reduktion der Noxenschädigung >80%; mit Positivkontrolle (Vaseline, Wollwachssalbe) vergleichbar

**Tabelle 9**

| Prüfsubstanz | **Exposition: 0,25h** | **Exposition: 1,0h** | **Exposition: 5,0h** |
|---|---|---|---|
| Toluol / Beispiel 11 | nicht bestimmt | gut (sehr gut) | Befriedigend (nicht ausreichend) |
| **Toluol / Saniwip®** | nicht bestimmt | gut (befriedigend) | nicht ausreichend (nicht ausreichend) |
| | | | |
| Toluol/ Beispiel 4 | nicht bestimmt | Befriedigend (wie Positivkontrolle) | Befriedigend (gut) |
| **Toluol/Saniwip®** | nicht bestimmt | nicht ausreichend (nicht ausreichend) | Befriedigend (nicht ausreichend) |
| | | | |
| Toluol/Beispiel 5 | nicht bestimmt | gut (befriedigend) | gut (befriedigend) |
| **Toluol/Stokolan®** | nicht bestimmt | nicht ausreichend (nicht ausreichend) | Nicht ausreichend (nicht ausreichend) |
| | | | |
| SDS/ Beispiel 11 | nicht bestimmt | Sehr gut (wie Positivkontrolle) | wie Positivkontrolle (gut) |
| **SDS / Saniwip®** | nicht bestimmt | sehr gut (sehr gut) | gut (sehr gut) |
| | | | |
| SDS / Beispiel 4 | nicht bestimmt | wie Positivkontrolle (wie Positivkontrolle) | wie Positivkontrolle (wie Positivkontrolle) |
| **SDS /Saniwip®** | nicht bestimmt | befriedigend (nicht ausreichend) | Befriedigend (nicht ausreichend) |
| | | | |
| SDS/ Beispiel 5 | nicht bestimmt | Gut (wie Positivkontrolle) | gut (sehr gut) |
| **SDS / Stokolan®** | nicht bestimmt | nicht ausreichend (nicht ausreichend) | Befriedigend (nicht ausreichend) |

In standardisierten Hautschutzprüfungen mit dem BUS-Modell (follikelhaltige / nicht-follikelhaltige Haut) wurden die Hautschutzpotentiale der Formulierungen Beispiel 11, Beispiel 4 und Beispiel 5 gegen Toluol (lipidlöslich) und SDS (10 % Aktivsubtsanz) im Vergleich zu Saniwip® bzw. Stokolan® untersucht. Die Einwirkzeit der Schutzprodukte war 15 Minuten, die Expositionszeit 0,25h, 1,0h und 5,0h. Die Formulierungen **Beispiel 11** und **Beispiel 5** erweisen sich am effektivsten. Ihre Schutzleistung entspricht in ca. 66 % der Hautschutzleistung der Positivkontrollen Vaseline oder Wollfettwachs. Eine gute Leistung weist auch die Formulierung **Beispiel 4** auf.

Eine geringere Schutzleistung als die genannten Formulierungen wird durch Saniwip® erreicht. Keine Schutzleistung gegen die Schädigung der Haut durch Toluol kommt durch die Applikation von Stokolan® zustande.

Gegen die durch SDS ausgelöste Hautschädigung wird eine den Positivkontrollen vergleichbare Schutzleistung durch Applikation der Formulierungen **Beispiel 11** und **Beispiel 4** erreicht. Nur unwesentlich geringer wirksam erweist sich die Schutzleistung der Formulierung Beispiel 5. Auch gegen die wasserlösliche Noxe SDS wird eine geringere Schutzleistung durch *Saniwip®* als durch die erfindungsgemäßen Formulierungen erreicht. Keine Schutzleistung kommt durch die Applikation von *Stokolan®* zustande.

## Patentansprüche

1. Kosmetische oder pharmazeutische O/W-Emulsion umfassend eine Ölphase und eine Wasserphase, wobei mindestens 40 Gew.-% der Ölphase eine Wachskomponente oder eine Gemisch aus Wachskomponenten ist.

2. Emulsion gemäß Anspruch 1, **dadurch gekennzeichnet, dass** der Anteil der Wachskomponente oder eines Gemisches aus Wachskomponenten bei 50 - 70 Gew.-%, vorzugsweise 55 - 70 Gew.-%, bezogen auf die Ölphase liegt.

3. Emulsion gemäß wenigstens einem der Ansprüche 1 bis 2, **dadurch gekennzeichnet, dass** wenigstens eine Wachskomponente einen Schmelzpunkt von wenigstens 30°C, vorzugsweise zwischen 40 - 80 °C aufweist.

4. Emulsion gemäß wenigstens einem der Ansprüche 1 bis 3 , **dadurch gekennzeichnet, dass** die Wachskomponente oder das Gemisch von Wachskomponenten ausgewählt ist aus der Gruppe der Fettalkohole, der gehärteten Mono-, Di- und Triglyceride oder einem Gemisch davon, der Fettsäuren, der natürliche pflanzlichen Wachse, der tierischen Wachse, der chemisch modifizierten Wachse, der synthetischen Wachse oder der Wachsester.

5. Emulsion gemäß wenigstens einem der Ansprüche 1 bis 4 , **dadurch gekennzeichnet, dass** als Wachskomponente(n) ein Wachsester in Kombination mit wenigstens einer weiteren Wachskomponente eingesetzt wird.

6. Emulsion gemäß wenigstens einem der Ansprüche 1 bis 5, **dadurch gekennzeichnet, dass** die Ölphase 15 - 35 Gew.-%, vorzugsweise 20 - 30 Gew.-%, bezogen auf die Gesamtzusammensetzung ausmacht.

7. Emulsion gemäß wenigstens einem der Ansprüche 1 bis 6, **dadurch gekennzeichnet, dass** die Ölphase wenigstens eine Ölkomponente ausgewählt aus der Gruppe der Dialkylcarbonate, der Triglyceride, der Dialklyether, der C12-C40-Kohlenwasserstoffe oder einem Gemisch dieser Substanzen enthält.

8. Emulsion gemäß wenigstens einem der Ansprüche 1 bis 7, **dadurch gekennzeichnet, dass** sie zusätzlich wenigstens einen grenzflächenaktiven Stoff aus der Gruppe der nichtionischen und/oder anionischen Tenside enthält.

9. Emulsion gemäß wenigstens einem der Ansprüche 1 bis 2, **dadurch gekennzeichnet, dass** die nichtionischen Tenside ausgewählt sind aus der Gruppe der Alk(en)yloligoglycoside.

10. Emulsion gemäß wenigstens einem der Ansprüche 1 bis 9 mit einem Gehalt an:
(a) 15 - 35 Gew.-% einer Ölphase, die 40 - 70 Gew.-% eines Gemisches and Wachskomponenten enthält, umfassend wenigstens C16-C24-Fettalkohole und Wachsester aus C12-C24-Fettalkoholen und C12-C24-Fettsäuren
(b) 0,5 - 5 Gew.-% eines nichtionischen Tensids ausgewählt aus der Gruppe der Alk(en)yloligoglycoside
(c) 50 - 80 Gew.-% Wasser
(d) und optional weitere Hilfs- und Zusatzstoffe.

11. Verwendung von Zusammensetzungen gemäß einem der Ansprüche 1 bis 10 zur Hautpflege und zum Hautschutz von Personen, die Kontakt mit Kühlschmierstoffen haben.
